# EUROPEAN PATENT APPLICATION

(11) **EP 4 623 753 A1**
(43) Date of publication of application: **01.10.2025**
(21) Application number: 24186689.6
(22) Date of filing: 04.07.2024
(51) Int. Cl.: A46B 15/00, A61B 5/06, A61B 5/00

(54) **SYSTEM AND METHOD FOR DETERMINING A LOCATION OF A PERSONAL CARE DEVICE**

(30) Priority: 28.03.2024 US 202463570888 P
(71) Applicant: Koninklijke Philips N.V., 5656 AG Eindhoven (NL)
(72) Inventor: VAN DEN DUNGEN, Wilhelmus Andreas Marinus Arnoldus Maria, Eindhoven (NL)
(74) Representative: Philips Intellectual Property & Standards

(57) **Abstract**

A system and method for determining a location of a personal care device that vibrates during use. A dataset describing an operating parameter of the personal care device during vibration of the personal care device is received and processed to generate a spectrogram representative of the dataset. A machine-learning algorithm is used to estimate a location of the personal care device based on the spectrogram.

## Description

### FIELD OF THE INVENTION

The invention relates to the field of personal care devices, and, in particular, to location tracking of personal care devices.

### BACKGROUND OF THE INVENTION

Location tracking is used in many personal care devices (such as toothbrushes) for a variety of purposes, such as analyzing use of the personal care device to provide guidance to a user, diagnostic purposes (to associate a detected issue with a location of the issue), and guiding treatment. For instance, location tracking of a toothbrush may be used to determine which areas of a user's mouth have and have not been brushed during a brushing session, and to provide feedback to the user guiding the user to brush any areas that have not yet been brushed. In another example, location tracking of a light treatment device may be used to control treatment settings of the light treatment device (e.g. to provide a desired level of light when the light treatment device is at a particular location within a user's mouth).

Location tracking in personal care devices typically relies on the use of expensive sensors, such as inertial measurement units (IMUs). This increases a cost and complexity of manufacturing a personal care device. Further, use of an IMU to determine location has limited accuracy.

### SUMMARY OF THE INVENTION

The invention is defined by the claims.

According to examples in accordance with an aspect of the invention, there is provided a processing system for determining a location of a personal care device, wherein the personal care device comprises an actuator that causes the personal care device to vibrate while in use, the processing system being configured to: receive a dataset describing an operating parameter of the personal care device during vibration of the personal care device; process the dataset to generate a spectrogram representative of the dataset; and process the spectrogram, using a trained machine-learning algorithm, to generate an estimate of the location of the personal care device.

The inventors have recognized that a vibration response of a personal care device that vibrates during use is characteristic of the location of the personal care device with respect to a user of the personal care device (e.g. a position within the mouth of a user, in the case of an oral care device), and that a machine-learning algorithm may be trained to classify a spectrogram of a dataset describing an operating parameter of the personal care device during vibration of the personal care device, based on features of the spectrogram, as belonging to a particular location.

The inventors have further recognized that a dataset describing an operating parameter of the personal care device during vibration of a personal care device may be acquired by sensors that are conventionally included in personal care devices, allowing the location of the personal care device to be determined without requiring an expensive position sensor such as an IMU.

In some examples, the machine-learning algorithm has been trained using a training algorithm configured to receive an array of training inputs and known outputs, each training input comprising a spectrogram representative of a dataset describing an operating parameter of a personal care device during vibration of the personal care device, and each known output comprising a location of the personal care device.

In some examples, the machine-learning algorithm is a convolutional neural network.

Convolutional neural networks have been found to be particularly advantageous for providing an accurate estimate of the location of a personal care device based on a spectrogram of a dataset describing an operating parameter of the personal care device during an operating parameter of the personal care device.

In some examples, the convolutional neural network may be a small convolutional neural network. For instance, the convolutional neural network may comprise fewer than 10 layers, e.g. 3-6 layers.

In some examples, the processing system is configured to process the dataset to generate the spectrogram by: normalizing the dataset; and processing the normalized dataset to generate the spectrogram.

Normalizing the dataset accounts for variations in the dataset due to product variation, thus improving a reliability of the estimated location of the personal care device.

In some examples, the dataset comprises values describing the operating parameter of the personal care device sampled at a rate of at least 800 Hz, and optionally at a rate of at least 1.6 kHz.

A sampling rate of at least 800 Hz improves a likelihood that the dataset captures unique features of the vibration response of the personal care device that relate to the location of the personal care device with respect to a user of the personal care device.

In some examples, the dataset comprises values describing the operating parameter of the personal care device spanning up to 1 second.

The inventors have recognized that the location of the personal care device can be determined using values acquired during a timespan of 1 second or less. A longer timespan may be used to acquire the values in the dataset if a higher level of accuracy is desired.

In some examples, the dataset comprises one or more of: acceleration data describing acceleration of the personal care device in at least one axis; audio data describing a sound produced by the personal care device; current data describing a motor current of the actuator of the personal care device and/or a battery current of a battery for the motor; and/or mechanical load data describing a mechanical load of the personal care device.

These types of data are responsive to the vibration response of a personal care device, and have been shown to be capable of providing sufficient information regarding vibration of the personal care device to enable the location of the personal care device to be determined.

In some examples, more than one type of data may be used to further improve the estimate of the location.

In some examples, the dataset comprises acceleration data acquired by a triple-axis accelerometer.

Acceleration data from a triple-axis accelerometer includes direction information, which may further improve an accuracy of the estimated location.

In some examples, the processing system is configured to process the dataset to generate the spectrogram by: concatenating information from each axis of the triple-axis accelerometer to produce a concatenated dataset; and processing the concatenated dataset to generate the spectrogram.

In this way, data relating to different directions can be distinguished in the spectrogram.

In some examples, the concatenated dataset is a normalized concatenated dataset, wherein information from each axis of the triple-axis accelerometer has been independently normalized.

This ensures that directional information is retained in the concatenated signal. In some examples, the concatenated dataset may further comprise a set of norm values, each norm value representing a magnitude of an acceleration measurement in the dataset.

In some examples, the personal care device further comprises an inertial measurement unit; and the processing system is further configured to: receive, from the inertial measurement unit, position data for the personal care device; and process the position data and the generated estimate of the location of the personal care device to determine a final estimate of the location of the personal care device.

Combining information from an inertial measurement unit and the output of the machine-learning algorithm may provide a more accurate estimate of the location than using either the inertial measurement unit alone or the output of the machine-learning algorithm alone.

There is also provided a personal care system, comprising: a personal care device comprising: an actuator capable of causing the personal care device to vibrate while in use; and at least one sensor capable of acquiring a dataset describing an operating parameter of the personal care device during vibration of the personal care device; and the processing system described above.

In some examples, the personal care device is an oral care device. For instance, the personal care device may be a toothbrush.

The estimated location of an oral care device may be an estimate of the location of the oral care device within the mouth of a user.

According to examples in accordance with another aspect of the invention, computer-implemented method for determining a location of a personal care device, wherein the personal care device comprises an actuator that causes the personal care device to vibrate while in use, the computer-implemented method comprising: receiving a dataset describing an operating parameter of the personal care device during vibration of the personal care device; processing the dataset to generate a spectrogram representative of the dataset; and processing the spectrogram, using a trained machine-learning algorithm, to generate an estimate of the location of the personal care device.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a personal care system, according to an embodiment of the invention;
Fig. 2 illustrates a personal care device of the personal care system;
Fig. 3 illustrates an example normalized concatenated waveform; and
Fig. 4 illustrates a computer-implemented method for determining a location of a personal care device, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for determining a location of a personal care device that vibrates during use. A dataset describing an operating parameter of the personal care device during vibration of the personal care device is received and processed to generate a spectrogram representative of the dataset. A machine-learning algorithm is used to estimate a location of the personal care device based on the spectrogram.

Embodiments are at least partly based on the realization that many personal care devices vibrate during use (either intentionally or as a side-effect of operation of the personal care device), and that vibrations of a personal care device are affected by the position of the personal care device with respect to a user. The inventors have further recognized that a machine-learning algorithm may be trained to identify features of a spectrogram representative of an operating parameter responsive to vibrations of the personal care device that are indicative of a location of the personal care device.

Illustrative embodiments may, for example, be employed in oral care devices, hair removal devices, and skin cleaning devices, and in applications for personal care devices.

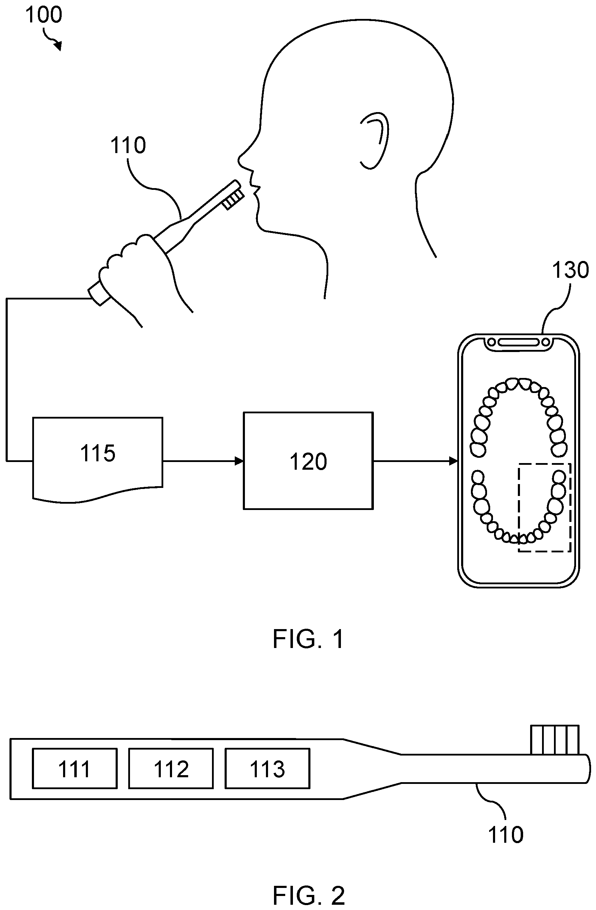

Fig. 1 illustrates a personal care system 100, according to an embodiment of the invention. The personal care system comprises a personal care device 110 and a processing system 120 for determining a location of the personal care device. The processing system 120 is, itself, an embodiment of the invention.

In Fig. 1, the personal care device 110 and the processing system 120 are illustrated as separate elements. However, in some examples, the processing system (or part of the processing system) may be provided in the personal care device. In other examples, the processing system may be provided in a device external to the personal care device; for instance, the processing system may be provided in a smartphone or tablet of a user of the personal care device.

The personal care device 110 is shown in more detail in Fig. 2. The personal care device comprises an actuator 111, the movement of which results in a vibrational response of the personal care device. The vibration of the personal care device caused by the actuator may or may not form part of the function of the personal care device (i.e. the vibration of the personal care device may form a function, or the vibration of the personal care device may be a side-effect of motion of the actuator for another purpose).

In Figs. 1 and 2, the personal care device 110 is an electric toothbrush, and the actuator is a motor configured to drive movement of a brush head of the electric toothbrush. However, the personal care device may be any kind of device used for personal care comprising an actuator that causes the personal care device to vibrate during use of the personal care device. For instance, the personal care device may be an electric shaver, an epilator, a skin scrubber, a body groomer, an oral irrigator or an ultrasonic tooth cleaner.

In some examples, the personal care device 110 may comprise at least one sensor 112 capable of acquiring a dataset describing an operating parameter of the personal care device during vibration of the personal care device. The operating parameter may be any operating parameter that is responsive to a vibration response of the personal care device. In other words, the at least one sensor may comprise any sensor capable of acquiring a dataset containing values indicative of a vibration response of the personal care device. The vibration response of the personal care device to movement of the actuator 111 is affected by the position of the personal care device with respect to a user of the personal care device.

In some examples, the at least one sensor 112 may comprise an accelerometer configured to acquire acceleration data for the personal care device 110. The accelerometer may, for example, be a triple-axis accelerometer; however, an accelerometer configured to acquire acceleration data in a single dimension provides sufficient data to obtain an estimate of the location of the personal care device.

In some examples, the at least one sensor 112 may comprise a microphone configured to measure sound produced by the personal care device 110.

In some examples, the at least one sensor 112 may comprise a current sensor configured to measure a current of a motor of the actuator 111 (i.e. a motor that drives movement of the actuator) or a battery to which the motor is connected.

In some examples, the at least one sensor 112 may comprise a load sensor configured to measure a mechanical load of the personal care device 110 (i.e. a force exerted by pressing the personal care device against a surface of the user, such as against the user's teeth, in the case of a toothbrush).

In some examples, the personal care device 110 may comprise an inertial measurement unit (IMU) 113. Data acquired by the IMU may be used to further improve an accuracy of the estimated location, as described in more detail below. In examples in which the personal care device comprises an IMU, an accelerometer of the IMU may be used to acquire the dataset describing an operating parameter of the personal care device during vibration of the personal care device.

Returning to Fig. 1, the processing system 120 is configured to receive a dataset 115 describing an operating parameter of the personal care device 110 during vibration of the personal care device 110. In the example of Figs. 1 and 2, the dataset 115 is acquired by the at least one sensor 112 of the personal care device. In other examples (e.g. where the personal care device does not include a suitable sensor for acquiring the dataset), the dataset may be acquired by at least one sensor external to the personal care device, such as a microphone provided in a charger base/dock for the personal care device or in a mobile device (e.g. a smartphone or a tablet). In some examples, the dataset may comprise data acquired by more than one sensor (i.e. the dataset may describe more than one operating parameter).

In Fig. 1, the dataset 115 is received by the processing system 120 directly from the at least one sensor 112. Alternatively, the dataset may be received from a memory unit storing the dataset.

As the skilled person will appreciate, the operating parameter(s) described by the dataset 115 will depend on the type(s) of sensor used to acquire the dataset. For instance, the dataset may comprise acceleration data describing an acceleration of the personal care device 110 in at least one axis, audio data describing a sound produced by the personal care device, current data describing a motor current of the actuator 111 of the personal care device and/or a battery current of a battery for the motor, and/or mechanical load data describing a mechanical load of the personal care device. Any other operating parameters suitable for capturing features indicative of vibration of the personal care device may additionally or alternatively be used.

The dataset 115 may comprise values sampled at a rate of at least 800 Hz. In some examples, the dataset may comprise values sampled at a rate of at least 1.6 kHz. A suitable sampling rate for the values in the dataset, i.e. in order to capture a frequency range containing unique features for location determination, may depend on a base frequency of the personal care device 110 and on the sensor used to acquire the values. For instance, for a personal care device having a base frequency of 260 Hz, acceleration data may be acquired at a sampling rate of 1.6 kHz, while audio data and/or current data may be acquired at a sampling rate of 16 kHz.

The bit depth (i.e. resolution) of values in the dataset 115 may also depend on the type of data. For instance, audio data may be captured at 16 bit, acceleration data may be captured at 12 bit, current data may be captured at 8 bit or 16 bit, while mechanical load data may be captured at 8 bit.

The dataset 115 may comprise values spanning a time of no more than a few seconds. For instance, the values may span a time of up to 1 second. For improved accuracy of location estimation, the values may be acquired over a longer period (e.g. up to 3 seconds). A suitable timespan for acquiring the values may depend on the sampling rate at which the values are acquired; for example, the values may be acquired over a longer period if the sampling rate is lower.

Having received the dataset, the processing system 120 is configured to process the dataset 115 to generate a spectrogram representative of the dataset, and to process the spectrogram, using a trained machine-learning algorithm, to generate an estimate of the location of the personal care device.

The generated spectrogram is an image that represents the signal strength of various frequencies in the vibration response of the personal care device 110 over the time spanned by the dataset. Suitable methods for generating the spectrogram, including the application of fast Fourier transforms (FFTs) to the dataset, will be readily apparent to the skilled person. For instance, spectrogram data may be generated by applying each of a plurality of FFTs to the dataset at a different point in time captured by the dataset, and an image representing the spectrogram data may then be generated from the spectrogram data.

In some examples, the generated spectrogram may be a low-resolution image (e.g. 128×128 pixels); a low-resolution spectrogram image has be shown to be sufficient for determining a location of a personal care device with high accuracy. In some examples, a higher resolution may be used for the spectrogram in order to further improve the accuracy of the location determination.

In some examples, processing the dataset 115 to generate the spectrogram may comprise a step of preprocessing the dataset. The preprocessed dataset may then be used to generate the spectrogram. For instance, the dataset may be normalized (e.g. to values between 0 and 1 or between -1 and +1), and the normalized dataset may be processed to generate the spectrogram. Normalizing the dataset may mitigate differences between individual personal care devices, by accounting for variations in amplitude (caused, for example, by production variations in the personal care device or differences in the environmental conditions in which the personal care device is being used).

As previously discussed, in some examples, the dataset 115 may comprise acceleration data acquired by a triple-axis accelerometer. Data acquired by a triple-axis accelerometer may be advantageous compared with data acquired by other types of sensor, as the data acquired by a triple-axis accelerometer includes directional information relating to the vibration response of the personal care device.

In order to distinguish acceleration values from each different axis within the spectrogram, where the dataset comprises acceleration data acquired by a triple-axis accelerometer, information from each axis of the triple-axis accelerometer may be concatenated or serialized to produce a concatenated dataset, and the concatenated dataset may be processed to generate the spectrogram.

In some examples, the concatenated dataset may further comprise a set of norm values. In other words, each acceleration measurement comprises 3 acceleration values (an X-axis value, a Y-axis value and a Z-axis value); a norm value may be determined for each acceleration measurement and included in the set of norm values in the concatenated dataset. The norm value for each acceleration measurement is a measure of the magnitude (i.e. absolute length) of the vector defined by the X-, Y- and Z-axis values for the acceleration measurement. The inclusion of norm values may further improve the performance of the location estimation, by providing additional information that may be used in the determination of the location.

In some examples, the concatenated dataset used to generated the spectrogram may be a normalized concatenated dataset, in which information from each axis of the triple-axis accelerometer (and, where present, the set of norm values) has been independently normalized. In other words, the normalized concatenated dataset may be produced by separately normalizing each set of acceleration values (i.e. the set of X-axis values, the set of Y-axis values, the set of Z-axis values, and, where present, the set of norm values). Independently normalizing each set of values ensures that directional information is preserved in the spectrogram.

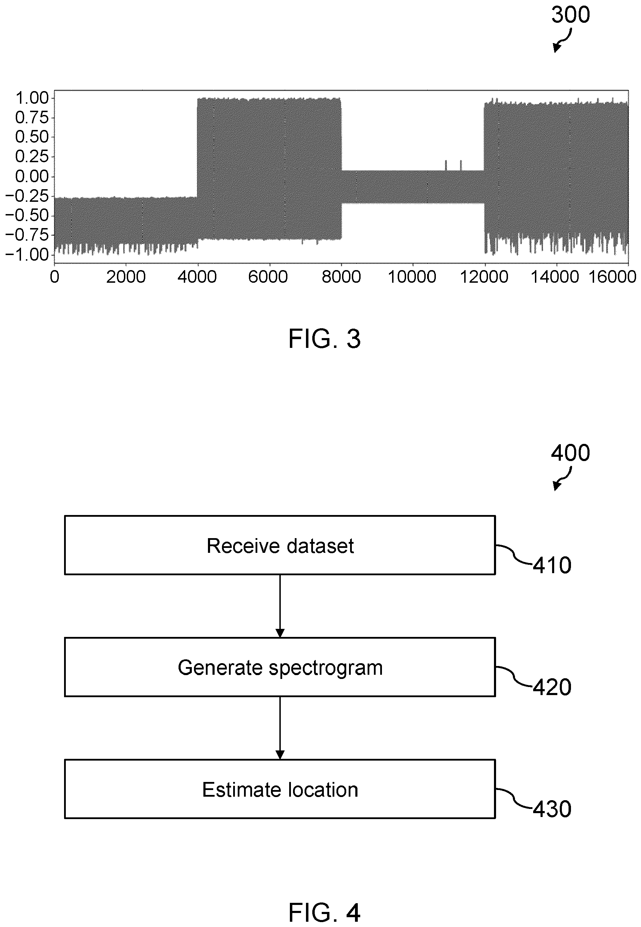

Fig. 3 illustrates an example normalized concatenated waveform 300, produced from a signal acquired by a triple-axis accelerometer over a period of 2.5 seconds at a sampling rate of 1.6 kHz. The normalized concatenated waveform 300 includes a set of norm values, resulting in a total of 16,000 data points (similar to a microphone sampling at a rate of 16 kHz for 1 second). The normalized concatenated waveform is provided in a XYZN format; in other words, the normalized concatenated waveform comprises a set of normalized X-axis values, followed by a set of normalized Y-axis values, a set of normalized Z-axis values and a set of normalized norm values. Each set of values has been independently normalized, as described above.

Similarly, in examples in which the dataset 115 comprises data acquired by more than one sensor (i.e. more than one type of sensor), the data acquired by each sensor may be concatenated in the dataset, and the spectrogram may be generated based on the concatenated dataset. The concatenated dataset may be a normalized concatenated dataset, with the data acquired by each sensor being normalized independently. Alternatively, a separate spectrogram may be generated for each sensor.

Returning to Fig. 1, the processing system 120 is, as stated above, configured to process the spectrogram, using a trained machine-learning algorithm, to generate an estimate of the location of the personal care device 110. In examples in which data is acquired by more than one sensor and a separate spectrogram is generated based on the data acquired by each sensor, each spectrogram may be processed using the trained machine-learning algorithm to generate an estimate of the location of the personal care device. The generated estimates of the location of the personal care device may then be processed to determine a final estimate for location of the personal care device.

A machine-learning algorithm is any self-training algorithm that processes input data in order to produce or predict output data. Here, the input data comprises a spectrogram representative of a dataset describing an operating parameter of the personal care device during vibration of the personal care device, and the output data comprises an estimate of the location of the personal care device with respect to a user of the personal care device.

Suitable machine-learning algorithms for being employed in the present invention will be apparent to the skilled person. Examples of suitable machine-learning algorithms include artificial neural networks and support vector machines.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs), fully connected neural networks (FCNNs) and recurrent neural networks (RNNs). Examples of the invention typically implement the machine-learning algorithm as a spectral image classifier, such as a CNN or an FCNN.

In particular, the machine-learning algorithm may be a classical convolutional neural network (CNN) classifier with a limited number of layers (e.g. 3-6 layers). It has been shown that a CNN with 3-6 convolutional layers provides effective processing of the spectrogram image. Further, these small CNN-based algorithms can easily be used in embedded firmware that may be present in existing personal care devices. Alternatively, as the sample sizes are small, cloud processing is also possible.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example spectrograms representative of a dataset describing an operating parameter of a personal care device during vibration of the personal care device. The training output data entries correspond to locations of the personal care device, each associated with an example spectrogram of the training input data.

The machine-learning algorithm may be (at least partially) pre-trained before deployment for use in determining a location of the personal care device during use by an end-user. In some examples, calibration and/or retraining may additionally be performed based on data obtained during use of the personal care device by an end-user of the device, for example, using an unsupervised learning method or by obtaining additional data during use by the end-user for verification. In this way, the trained machine-learning algorithm may be personalized to the end-user.

The training data may comprise data obtained from real user tests and/or synthetic data. Data obtained from real user tests may be obtained by acquiring a dataset describing an operating parameter (e.g. acceleration, sound, current and/or mechanical load) as a (human) user uses the personal care device, and simultaneously acquiring separate location data for the personal care device (e.g. using an inertial measurement unit). Synthetic data may be obtained by acquiring a dataset describing an operating parameter as a robotic testing setup uses the personal care device, and simultaneously acquiring separate location data for the personal care device. Alternatively, synthetic data set may be generated using a computer simulation. The use of synthetic data may enable the generation of large amounts of training data, spanning a wide variety of test conditions, in a relatively cheap and quick manner (compared to data obtained from human user operation). In turn, by providing more training data, an accuracy and efficacy of the machine-learning algorithm may be improved.

In some examples, the machine-learning algorithm may be trained in a way that takes into account variations in grip of the personal care device according to location. Users of personal care devices grip a personal care device in different ways depending on the position of the personal care device with respect to the user, and differences in grip (as well as changes in grip) influence the vibration response of the personal care device. The machine-learning algorithm may be trained to identify a vibrational response associated with a particular grip as indicative of a location in which that grip is used, and to identify a change in vibrational response associated with a change in grip as indicative of a change of location of the personal care device. For instance, where the training data includes synthetic data, the synthetic data may be generated in a way that includes the relationship between grip and location (e.g. by using realistic grips for a robotic testing setup, and changing the grip when changing location as appropriate).

The machine-learning algorithm may be trained to estimate the location of the personal care device 110 to a desired resolution/level of detail. For instance, in the case of an oral care device, the machine-learning algorithm may be trained to estimate whether the oral care device is by an upper or lower arch of teeth. If a higher resolution is desired, the machine-learning algorithm may be trained to estimate which tooth quadrant the oral care device is in (e.g. upper left, upper right, lower left or lower right). The estimate may relate to an even smaller area of the mouth (e.g. one of upper left front, upper left rear, upper right front, upper right rear, lower left front, lower left rear, lower right front, lower right rear) if a still higher resolution is desired. For personal care devices used on a user's skin, the machine-learning algorithm may be trained to estimate which body part the personal care device is being used, or a particular location with respect to the body part if a higher resolution is desired.

The machine-learning algorithm may also be trained to detect that a location of the personal care device is outside a target area (e.g. when the personal care device is not inside the mouth of a user in the case of an oral care device, or when a hair-removing element of the personal care device is not in contact with the user in the case of a hair-removal device).

In some examples, the machine-learning algorithm may generate a probability score for each of a plurality of possible locations for the personal care device, the probability score for a location representing a probability that the personal care device is at the location. The estimate of the location of the personal care device may then be generated based on the probability scores (e.g. the location corresponding to the highest probability score may be selected as the estimated location of the personal care device).

As previously mentioned, in some examples, the personal care device 110 may comprise an inertial measurement unit (IMU) 113. In these examples, the processing system 120 may be configured to receive position data acquired by the IMU, and the generated estimate of the location of the personal care device may be further based on the position data acquired by the IMU. This may provide a more accurate estimate of the location of the personal care device than using IMU data alone.

Any suitable sensor fusion approach may be used to generate an estimate of the location of the personal care device that is based both on the vibrational response of the personal care device and on position data acquired by the IMU. For instance, the position data acquired by the IMU may be concatenated with the dataset 115 before generating the spectrogram, so that the spectrogram input to the machine-learning algorithm includes information from the IMU as well as information relating to the vibrational response of the personal care device. Alternatively, the position data acquired by the IMU may be processed to obtain a separate estimate of the location of the personal care device, which may be combined with the estimate output by the machine-learning algorithm (i.e. based on the vibrational response) to determine a final estimate for the location of the personal care device.

In some examples, the personal care system 100 may further comprise an output user interface 130. The processing system 120 may be configured to control the output user interface to provide a user-perceptible output representative of the estimated location of the personal care device 110 (e.g. a visual representation of the estimated location).

In Fig. 1, the output user interface 130 is a smartphone, and the user-perceptible output comprises an image indicating the tooth quadrant in which the personal care device is estimated to be. Further suitable output user interfaces and/or user-perceptible outputs will be apparent to the skilled person.

The processing system 120 may additionally or alternatively be configured to use the estimated location of the personal care device 110 to control an operation of the personal care device. For instance, in the case of a hair removal device, the processing system may control a setting of the hair removal device according to the body part the hair removal device is being used on. In the case of a dental light therapy device, the processing system may control a light setting of the light therapy device according to the location of the light therapy device within the user's mouth.

In some examples, the personal care device 110 may comprise at least one sensor for making a diagnosis of a disease or condition (e.g. for detecting a skin condition in the case of a personal care device used on a user's skin, or for detecting tooth decay or a gum disease in the case of an oral care device). For instance, the at least one sensor for making a diagnosis of a disease or condition may comprise a camera. Other suitable sensors may depend on the personal care device and the disease or condition to be diagnosed.

The processing system 120 may be configured to receive data from the at least one sensor for making a diagnosis of a disease or condition, and to associate the received data with the estimated location of the personal care device 110 at which the data was acquired. In this way, a location of a health issue may be determined.

Fig. 4 illustrates a computer-implemented method 400 for determining a location of a personal care device, according to an embodiment of the invention. The method 400 may be used to determine a location of any personal care device that comprises an actuator that causes the personal care device to vibrate while in use.

The computer-implemented method 400 begins at step 410, at which a dataset is received. The dataset describes an operating parameter of the personal care device during vibration of the personal care device. The operating parameter may be any parameter responsive to a vibration response of the personal care device, such as acceleration, sound, current and/or mechanical load, as described above.

At step 420, the dataset is processed to generate a spectrogram representative of the dataset. In some examples, step 420 may comprise a sub-step of preprocessing the dataset before generating the spectrogram. For instance, the dataset may be normalized, as described above.

At step 430, the spectrogram is processed, using a trained machine-learning algorithm, to generate an estimate of the location of the personal care device. The machine-learning algorithm may be trained as described above. In some examples, the machine-learning algorithm may be a convolutional neural network (e.g. having 3-6 layers).

In some examples, the personal care device may comprise an inertial measurement unit, and the computer-implemented method may further comprise a step (not shown in Fig. 4) of receiving position data from the inertial measurement unit, and processing the position data and the generated estimate of the location of the personal care device to determine a final estimate of the location of the personal care device.

In some examples, the computer-implemented method may further comprise a step (not shown in Fig. 4) of controlling an output user interface to provide a user-perceptible output representative of the estimated location of the personal care device.

In some examples, the computer-implemented method may further comprise a step (not shown in Fig. 4) of controlling an operation of the personal care device based on the estimated location of the personal care device.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A processing system (120) for determining a location of a personal care device (110), wherein the personal care device comprises an actuator (111) that causes the personal care device to vibrate while in use, the processing system being configured to:
receive a dataset (115) describing an operating parameter of the personal care device during vibration of the personal care device;
process the dataset to generate a spectrogram representative of the dataset; and
process the spectrogram, using a trained machine-learning algorithm, to generate an estimate of the location of the personal care device.

2. The processing system (120) of claim 1, wherein the machine-learning algorithm has been trained using a training algorithm configured to receive an array of training inputs and known outputs, each training input comprising a spectrogram representative of a dataset describing an operating parameter of a personal care device during vibration of the personal care device, and each known output comprising a location of the personal care device.

3. The processing system (120) of claim 1 or 2, wherein the machine-learning algorithm is a convolutional neural network.

4. The processing system (120) of any of claims 1 to 3, wherein the processing system is configured to process the dataset (115) to generate the spectrogram by:
normalizing the dataset; and
processing the normalized dataset to generate the spectrogram.

5. The processing system (120) of any of claims 1 to 4, wherein the dataset (115) comprises values describing the operating parameter of the personal care device (110) sampled at a rate of at least 800 Hz, and optionally at a rate of at least 1.6 kHz.

6. The processing system (120) of any of claims 1 to 5, wherein the dataset (115) comprises values describing the operating parameter of the personal care device (110) spanning up to 1 second.

7. The processing system (120) of any of claims 1 to 6, wherein the dataset (115) comprises one or more of:
Acceleration data describing acceleration of the personal care device (110) in at least one axis;
audio data describing a sound produced by the personal care device;
current data describing a motor current of the actuator of the personal care device and/or a battery current of a battery for the motor; and/or
mechanical load data describing a mechanical load of the personal care device.

8. The processing system (120) of claim 7, wherein the dataset (115) comprises acceleration data acquired by a triple-axis accelerometer.

9. The processing system (120) of claim 8, wherein the processing system is configured to process the dataset (115) to generate the spectrogram by:
concatenating information from each axis of the triple-axis accelerometer to produce a concatenated dataset; and
processing the concatenated dataset to generate the spectrogram.

10. The processing system (120) of claim 9, wherein the concatenated dataset is a normalized concatenated dataset, wherein information from each axis of the triple-axis accelerometer has been independently normalized.

11. The processing system (120) of any of claims 1 to 10, wherein:
the personal care device (110) further comprises an inertial measurement unit (113);
the processing system is further configured to receive, from the inertial measurement unit, position data for the personal care device; and
the generated estimate of the location of the personal care device is further based on the position data received from the inertial measurement unit.

12. A personal care system (100), comprising:
a personal care device (110) comprising:
an actuator (111) capable of causing the personal care device to vibrate while in use; and
at least one sensor (112) capable of acquiring a dataset describing an operating parameter of the personal care device during vibration of the personal care device; and
the processing system (120) of any of claims 1 to 11.

13. The personal care system (100) of claim 12, wherein the personal care device (110) is an oral care device.

14. A computer-implemented method (400) for determining a location of a personal care device, wherein the personal care device comprises an actuator that causes the personal care device to vibrate while in use, the computer-implemented method comprising:
receiving a dataset describing an operating parameter of the personal care device during vibration of the personal care device;
processing the dataset to generate a spectrogram representative of the dataset; and
processing the spectrogram, using a trained machine-learning algorithm, to generate an estimate of the location of the personal care device.

15. A computer program product comprising computer program code means which, when executed on a computing device having a processing system, cause the processing system to perform all of the steps of the method (400) according to claim 14.
